# EUROPEAN PATENT SPECIFICATION

(11) **EP 1 194 158 B1**
(45) Date of publication and mention of the grant of the patent: **28.01.2004**
(21) Application number: 00936893.7
(22) Date of filing: 19.06.2000
(51) Int. Cl.: A61K 38/17, A61P 37/06

(54) **THE USE OF THE PROTEIN UK 114 FOR INHIBITING ORGAN TRANSPLANT REJECTION**
VERWENDUNG DES PROTEINS UK 114 ZUR VERHINDERUNG DER ABSTOSSUNG EINES TRANSPLANTIERTEN ORGANS
UTILISATION DE LA PROTEINE UK 114 PERMETTANT D'INHIBER LE REJET D'ORGANES GREFFES

(30) Priority: 22.06.1999 IT MI991384
(43) Date of publication of application: 10.04.2002
(73) Proprietor: ZETESIS S.P.A., 20122 Milano (IT)
(72) Inventor: BARTORELLI, Alberto, I-20122 Milano (IT); PANERAI, Alberto, I-20122 Milano (IT); NICOLETTI, Pierferdinando, I-20122 Milano (IT)
(74) Representative: Minoja, Fabrizio, Dr.
(86) International application number: PCT/EP2000/005621
(87) International publication number: WO 2000/078329

(56) References cited:
- WO-A-99/43340
- BUSSOLATI G ET AL: "CYTOLYTIC AND TUMOR INHIBITORY ANTIBODIES AGAINST UK114 PROTEIN IN THE SERA OF CANCER PATIENTS" INTERNATIONAL JOURNAL OF ONCOLOGY,GR,EDITORIAL ACADEMY OF THE INTERNATIONAL JOURNAL OF ONCOLOGY,, vol. 10, no. 4, 1 April 1997 (1997-04-01), pages 779-785, XP002040514 ISSN: 1019-6439
- CECILIANI F ET AL: "THE PRIMARY STRUCTURE OF UK114 TUMOR ANTIGEN" FEBS LETTERS,NL,ELSEVIER SCIENCE PUBLISHERS, AMSTERDAM, vol. 393, 16 September 1996 (1996-09-16), pages 147-150, XP002040513 ISSN: 0014-5793
- PANERAI ALBERTO E ET AL: "Chronic administration of UK-114, a multifunctional emerging protein, modulates the Th1/Th2 cytokine pattern and experimental autoimmune diseases." ANNALS OF THE NEW YORK ACADEMY OF SCIENCES, vol. 876, 22 June 1999 (1999-06-22), pages 229-235, XP000971426 First International Conference on the Neuroendocrine Immune Basis of the Rheumatic Diseases;Genova, Italy; September 18-20, 1998, basis of the rheumatic diseases. June 22, 1999 New York Academy of Sciences 2 East 63rd Street, New York, New York 10021, USA ISBN: 1-57331-215-0

## Description

The present invention relates to the use of the protein UK 114 for the preparation of a medicament for reducing or inhibiting the rejection of organ transplants, as well as for maintaining transplant acceptance.

At present the continuous evolution of surgery techniques, together with the development of rather selective, more effective immunosuppressive drugs, have progressively improved the outcome of organ transplants.

However, organ transplantation as permanent solution in case of end-stage organ failure still suffers in practice from the severe problem of the rejection reaction. Treatment with immunosuppressive drugs is used to control such problem, with the consequence that infective complications are still the leading cause of death in transplant recipients. Furthermore, immunosuppressive therapy can never be stopped completely, even after resolution of any acute rejection, but it has to be continued as maintenance treatment indefinitely, although with relatively small doses.

The rejection of allografts (between genetically dissimilar members of the same species) can take place due to either a cell-mediated or a humoral immune reaction against the histocompatibility antigens (HLA) present on the membranes of the donor's cells.

The cell-mediated immune reaction causes graft destruction days to months after transplantation (acute rejection) and is characterized by progressive infiltration of mononuclear cells (macrophages, lymphocytes and monocytes) in the transplanted tissue; if these cells perceive antigen differences, they will activate T lymphocytes, which stimulate an immune response, both cellular (T cells) and humoral (B cells) type, causing the destruction of the transplant. This type of cell-mediated rejection can often be treated with strong immunosuppressive therapy. In case of resolution, a novel acute rejection is unlikely to take place, and the allograft will usually survive for prolonged periods.

The role of humoral antibodies in rejection is clear when the recipient has been presensitized to the HLA antigens present in the graft: in these cases destruction of the transplanted organ takes place within a few hours or even minutes after the revascularization **(hyperacute rejection).**

**Chronic rejection** is a gradual process of deterioration and failure that occurs later in the life of the transplant, from several months to many years. The immunological mechanisms of the chronic rejection are less clear. The histological picture is different from that of acute rejection, and is characterized by lesions mainly on the arterial endothelium, where extensive proliferation gradually causes occlusion of the vessel lumen, ischemia and fibrosis of the graft.

Immunosuppressive treatment to control organ rejection is at present based on the use of corticosteroids, azathioprine (or cyclophosphamide in case of patients who do not tolerate azathioprine) and cyclosporin, often in a combination thereof.

However, each one of these medicaments involves a number of undesired side effects, which can be summarized as follows:
- corticosteroids: diabetogenicity, increase in proteins catabolism, adrenal cortex atrophy, reduction of the response of connective tissue to lesions, myopathy, osteoporosis, effects on the hematopoietic system and on nervous system);
- azathioprine: depression of bone marrow, hepatitis;
- cyclophosphamide: nefrotoxicity;
- cyclosporin: nefrotoxicity, hepatotoxicity, refractory hypertension and increase in neoplasias.

It is therefore evident the need for an alternative therapy preventing or reducing the rejection of a transplanted organ or tissue, or which anyway increases acceptance by the receiver without involving the above mentioned side effects.

It has now been found, and this is the object of the invention, that the protein having molecular weight of about 14 kDa in SDS-PAGE and obtainable by extraction from mammal liver with perchloric acid, disclosed in WO 97/30154 and in WO 96/02567 and known under the name UK 11.4, is capable of reducing or preventing rejection in allografts as well as maintaining the acceptance of the graft itself, without inducing the side effects typical of known immunosuppressive drugs.

The protein UK 114 acts on the immune system, exerting pleiotropic effects presumably due to a modulation of the cytokine production by T cells and macrophages.

According to the invention, the protein UK 114 of either extractive or recombinant origin can be administered parenterally, for example through the intramuscular, intravenous, intraperitoneal, subcutaneous, or sublingual routes.

Preferred formulation forms will be injectables forms, such as solutions or suspensions, sterile powders for the preparation of injectable solutions or suspensions; or solid forms such as tablets for the sublingual administration.

The protein of the invention may optionally be administered in combination with other conventional immunosuppressors, such as corticosteroids, azathioprine, cyclophosphamide, cyclosporin, also in combination thereof.

The dose to be administered will depend on a number of factors, such as the individual characteristics of the patient (weight, etc.) as well as on the type of organ or tissue transplanted or intended for transplantation. In general, however, the amount of UK 114 to be administered will vary from 0.1 to 30 mg/kg/day for a time of 1 to 6 months after transplantation. After that, a maintenance treatment will be followed.

The administration procedures, such as dosage, administration route, duration of the attack and maintenance treatments, possible administration of known immunosuppressive or chemotherapic agents, either concomitantly or separately, will be defined by the skilled clinician.

The following example further illustrates the invention.

### Example

Pancreatic islets transplantation in the mouse.

400 to 500 pancreatic islets were prepared from 5-6 week old euglycemic NOD mice, available from Charles River (Calco, Italy), and implanted under the renal capsule of female NOD mice suffering from spontaneous diabetes of recent onset (7-4 days) according to the procedure described by Mellgren A. et al., in Diabetologia, 1986, 29:670. This procedure allows to restore normoglycemia in NOD mice, followed by reappearance of hyperglycemia within 6-8 days, due to the destruction of the transplanted islets (see Sandberg J.O. et al., Clin. Exp. Immunol., 1997, 108:314).

### Treatment with UK 114

Starting two days before transplantation, and subsequently every day for the duration of the experiment, three groups of 2-25 week old diabetic NOD mice (15 animals/group) were treated intraperitoneally with UK 114 at a dose of 30 or 60 µg/mouse (in 0.1 or 0.2 ml) or with 0.2 ml of PBS. Glycemia was measured in animals on days 3, 6, 9, 12 and 14 after transplantation from tail blood samples (ExacTech, Baxter Travenol, Deerfield, IL). The animals were considered diabetic when glycemia was higher than 11.8 mmol/l after 6 hour fast.

During the experiment one animal of the control group and another of the group treated with UK 114 at low dosage died on day 2 and 3 after transplantation and they were not considered in the calculation of data.

On day 3 of the experiment, 5/14 mice of the PBS-treated control group were still normoglycemic, on day 6 only one mouse of this group was normoglycemic, and on day 9 all animals were markedly hyperglycemic with glycemia mean values (SD) of 16.2 ± 3.2 mmol/l, which further increased to 18.9 ± 4.5 on day 14 (See table).

Conversely, treatment with UK 114 dose-dependently prevented reappearance of hyperglicemia in transplanted NOD mice. Of the animals treated with 30 µg of UK 114, 12/14 animals were normoglycemic on day 6 and 11 out of 14 on day 9 and 14 (See table). None of the 15 animals treated with the higher dosage of UK 114 became diabetic during the 14 days subsequent transplantation (See table).

These data prove that the administration of UK 114 can prevent, or anyhow delay, rejection of pancreatic islets in a well-known animal model and suggest the potential use of the treatment with UK 114 in the prevention of the rejection of transplanted pancreatic islets in IDDM patients.

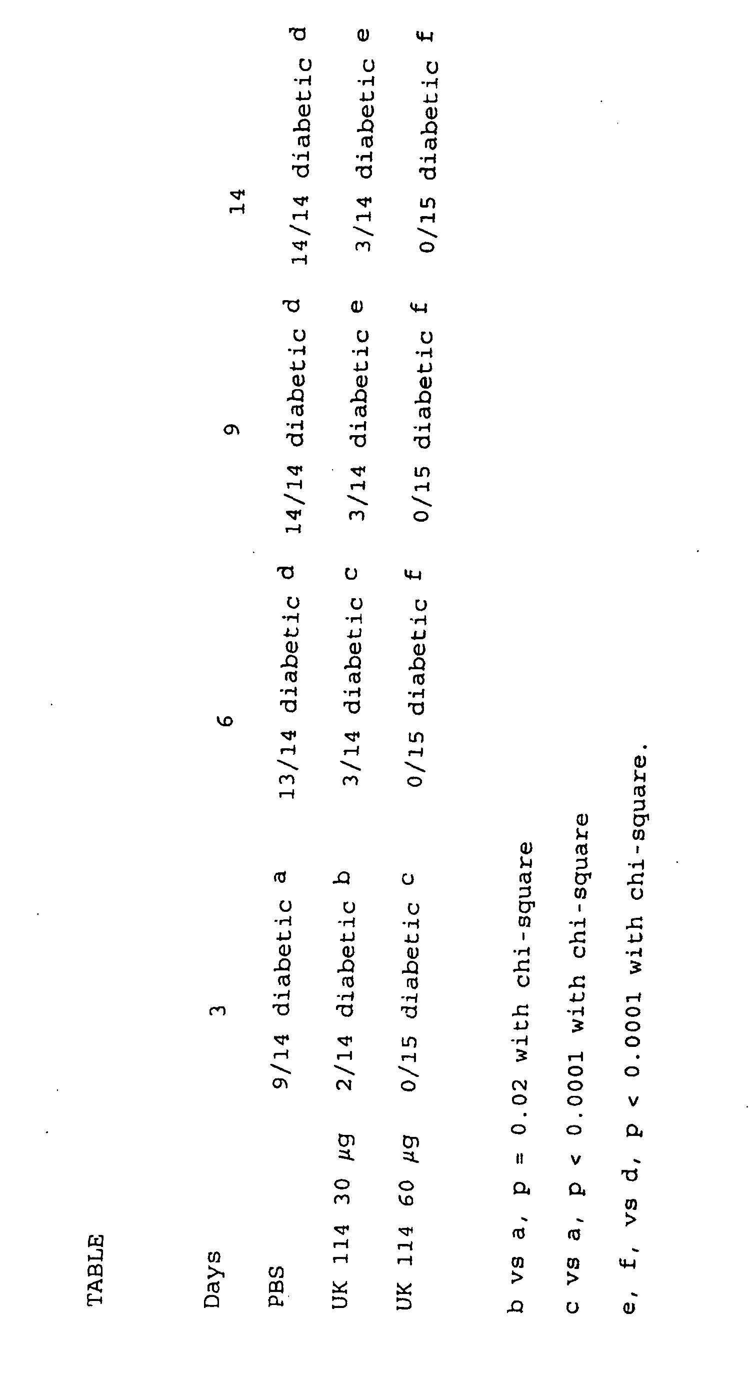

## Claims

1. The use of the protein UK 114 for the preparation of α medicament for reducing or inhibiting the rejection of organ transplants and for maintaining the acceptance of the transplant itself.

2. The use as claimed in claim 1, for reducing or preventing the rejection of Langerhans cells transplants.

## Patentansprüche

1. Verwendung des Proteins UK 114 für die Herstellung eines Medikamentes zur Verringerung bzw. Inhibierung des Abstoßens bei Organtransplantationen und zur Aufrechterhaltung der Annahme des Transplantats selbst.

2. Verwendung nach Anspruch 1, zur Verringerung bzw. Prävention des Abstoßens des Langerhons-Zellentransplantats.

## Revendications

1. Utilisation de la protéine UK 114 pour la préparation d'un médicament destiné à réduire ou inhiber le rejet des greffes d'organes et à maintenir l'acceptation de la greffe elle-même.

2. Utilisation selon la revendication 1, pour la réduction ou la prévention du rejet des greffes de cellules de Langerhans.
